# EUROPÄISCHE PATENTSCHRIFT

(11) **EP 1 593 622 B1**
(45) Veröffentlichungstag und Bekanntmachung des Hinweises auf die Patenterteilung: **29.07.2009**
(21) Anmeldenummer: 04405712.3
(22) Anmeldetag: 17.11.2004
(51) Int. Cl.: B65G 69/18

(54) **Transportbehälter für sterile Produkte**
Transport container for sterile products
Conteneur de transport pour produits steriles

(30) Priorität: 04.05.2004 CH 7882004
(43) Veröffentlichungstag der Anmeldung: 09.11.2005
(73) Patentinhaber: Netzhammer, Eric, 4103 Bottmingen (CH)
(72) Erfinder: Netzhammer, Erich, 4132 Muttenz (CH)
(74) Vertreter: Zbinden, Paul A.

(56) Entgegenhaltungen:
- EP-A- 1 253 095
- WO-A-98/43902
- US-A- 5 490 546
- US-B1- 6 305 443

## Beschreibung

Die vorliegende Erfindung betrifft einen Transportbehälter für den Transport steriler Produkte von einer Sterilisationseinrichtung zu einer nachgeordneten Einrichtung für die bestimmungsgemässe Verwendung der Produkte, sowie das sterile Andocken an die nachgeordnete Einrichtung, mit zwei einander gegenüber angeordneten Anschlussvorrichtungen, von denen eine mit einem Rapid Transfer Port zum Andocken des Transportbehälters an die nachgeordnete Einrichtung ausgerüstet ist und die andere eine Einfüllvorrichtung ist.

Bei den Produkten handelt es sich beispielsweise um Kleinteile wie Verschlusskappen oder -stopfen für pharmazeutische Verwendungen. Die nachgeordnete Einrichtung ist beispielsweise eine Abfüllanlage für pharmazeutische Produkte. Somit muss beim Andocken des Transportbehälters der Reinheitsgrad und die Sterilität der Produkte, sowie der Teile, mit welchen die Produkte in Berührung kommen, gewährleistet sein.

Für das Reinigen und Sterilisieren von Verschlusskappen oder -stopfen in der pharmazeutischen Industrie sind verschiedene Verfahren für die Behandlung und verschiedenen Methoden für das Andocken an die nächste Produktionsstufe bekannt.

Ein übliches Verfahren besteht darin, die Verschlussteile in einer Behandlungseinrichtung zu reinigen und zu sterilisieren und nach der Behandlung in einen Transport- und Lagerbehälter abzufüllen. Anschliessend wird der gefüllte Behälter von der Behandlungseinrichtung abgedockt. Der Behälter wird üblicherweise mit Druck überlagert, so dass eine Kontamination der Verschlussteile aus der Atmosphäre ausgeschlossen ist.

Bei Bedarf werden die Behälter zum nächsten Prozessschritt z.B. eine Abfüllmaschine gefahren. Normalerweise findet die weitere Verarbeitung in einem Reinraum oder in einem Isolator statt. Dort findet ein Andockvorgang statt. Entscheidend ist dabei, dass die Verschlussteile bei der weiteren Verarbeitung nie in Kontakt mit einer unreinen, d.h. einer nicht gereinigten und nicht sterilen Umgebung kommen. Deshalb müssen sämtliche Andockvorgänge unter sterilen Bedingungen erfolgen, so dass keine Kontamination der Verschlussteile mit der Atmosphäre stattfinden kann. Der Behälter hat in diesem Fall nur die Funktion eines Lager- und Transportbehälters. Es findet keine Behandlung des Inhalts darin statt.

Nach dem Einfüllen der behandelten Verschlussteile in den Transport- und Lagerbehälter wird manuell ein an sich bekannter sog. Rapid Transfer Port auf der Einfüllöffnung befestigt. Das dabei aufgesetzte Zwischenstück muss anschliessend mit den bekannten Mitteln gereinigt und sterilisiert werden. Nach diesem Prozess ist der Lagerbehälter bereit für das Andocken an die nächste Prozessstufe. Diese Methode hat den Nachteil, dass erstens mit dem Aufsetzen des Ports eine manuelle Operation erforderlich ist und dass zweitens die Reinigung und Sterilisierung des Ports nicht gleichzeitig mit dem Prozess durchgeführt werden kann.

Ein anderes bekanntes Verfahren besteht darin, die Verschlussteile in einem multifunktionalen Behälter zu behandeln und anschliessend in diesem Behälter zu lagern und zu transportieren.

Aus der unter Art. 54(3) EPÜ fallenden EP-A-1 510 227 ist ein Behandlungs- und Transportbehälter zur Reinigung, Sterilisation und Transport von kleinen Gegenständen bekannt, welcher zwei einander gegenüber angeordnete Anschlussvorrichtungen aufweist, von denen eine mit einem Rapid Transfer Port ausgerüstet ist und sowohl zum Befüllen mit den zu behandelnden Gegenständen, als auch zu deren Entleeren in eine nachgeordnete Einrichtung dient, während die andere für die Zuführung eines Behandlungsmediums vorgesehen ist.

Aus der WO-A-00/74735 ist ein Behandlungsbehälter zum Sterilisieren von Gegenständen mit einander gegenüber angeordneten Anschlussvorrichtungen bekannt, von denen eine mit einer Y-förmigen Verzweigung versehen ist. Von den beiden Armen der Verzweigung dient einer der Behandlung der im Behälter befindlichen Gegenstände und der andere der Befüllung mit den zu behandelnden Gegenständen und deren Entleerung. Der gegenüberliegende Anschluss ist für eine weitere Verbindung mit einer Behandlungsstation vorgesehen. Der Behälter der WO-A-00/74735 entspricht dem Oberbegriff des Anspruchs.

Um gute Reinigungs- und Sterilisationsergebnisse zu erzielen, sind grosse Mengen von Reinigungsmittel, Luft zur Trocknung und Dampf zur Sterilisation erforderlich. Diese Medien müssen in der Regel von unten nach oben durch das Gut strömen. Deshalb benötigt der multifunktionale Behälter einen oberen und einen unteren Prozessanschluss damit die erforderlichen Medien durch das Gut strömen können. Anschliessend an die Behandlung wird der multifunktionale Behälter von der Behandlungseinrichtung abgedockt. Die Verschlussteile werden im multifunktionalen Behälter unter Oberdruck transportiert und gelagert.

Für die weitere Verwendung wird wie beim ersten Verfahren vorgegangen. Um sowohl die Reinigungs- und Sterilisationsbehandlung als auch das Andocken in der nachfolgenden Prozessstufe zu ermöglichen, ist der multifunktionale Behälter oben mit einem Y-Anschluss ausgerüstet. Der eine Anschluss dient der Prozessdurchführung, der andere ist mit einem Rapid Transfer Port ausgerüstet. Mit dieser Einrichtung ist es möglich den Rapid Transfer Port fest an den Behälter zu montieren. Ein manuelles Aufsetzen des Port entfällt. Der Port ist mit einem Deckel ausgerüstet. Nachteilig bei dieser Methode ist, dass die Medien (Reinigungsmittel, Dampf) nicht durch den Port durchströmen können, was bakteriologisch nachteilig ist, da es gute Praxis ist, dass die produktberührten Teile bei der Reinigung und bei der Sterilisation vollständig mit Medium durchströmt werden sollten.

Ein zusätzlicher Nachteil ist der Umstand, dass die Dichtung des Port nicht gereinigt und nicht sterilisiert wird, da der Portdeckel beim Reinigungs- und Sterilisierprozess nicht entfernt wird. Zudem ist die Konstruktion des Y-Anschlusses sehr aufwendig. Diese Konstruktion ist jedoch bei diesem Verfahren notwendig, da die Prozessanschlüsse nicht als Portanschlüsse verwendet werden können.

Beim Andockprozess fällt das Gut nach unten und es muss mechanisch (in diesem Fall mittels Sieb) verhindert werden, dass das Gut in Richtung Prozessanschluss fällt.

Aus US-A-5,490,546 ist ein Transportbehälter mit einer an seiner Unterseite angeordneten Auslassöffnung und einer gegenüber an der Oberseite angeordneten Einfüllöffnung für sterile oder gefährliche Produkte bekannt. Beide Öffnungen sind mit Verschlussklappen versehen, die beim Andocken an eine Leitung, einen anderen Behälter oder dergl. durch dort vorhandene Betätigungsvorrichtungen geöffnet werden. Dadurch besteht auch bei diesem Transportbehälter der vorstehend erwähnte Nachteil hinsichtlich der unvollständigen Reinigungs- und Sterilisierungsmöglichkeit.

Im Hinblick auf die Nachteile der im Stand der Technik bekannten Systeme liegt der Erfindung die Aufgabe zugrunde, eine Lösung zu suchen, bei der diese Nachteile nicht vorhanden sind.

Diese Aufgabe wird durch die Merkmale des Anspruchs gelöst.

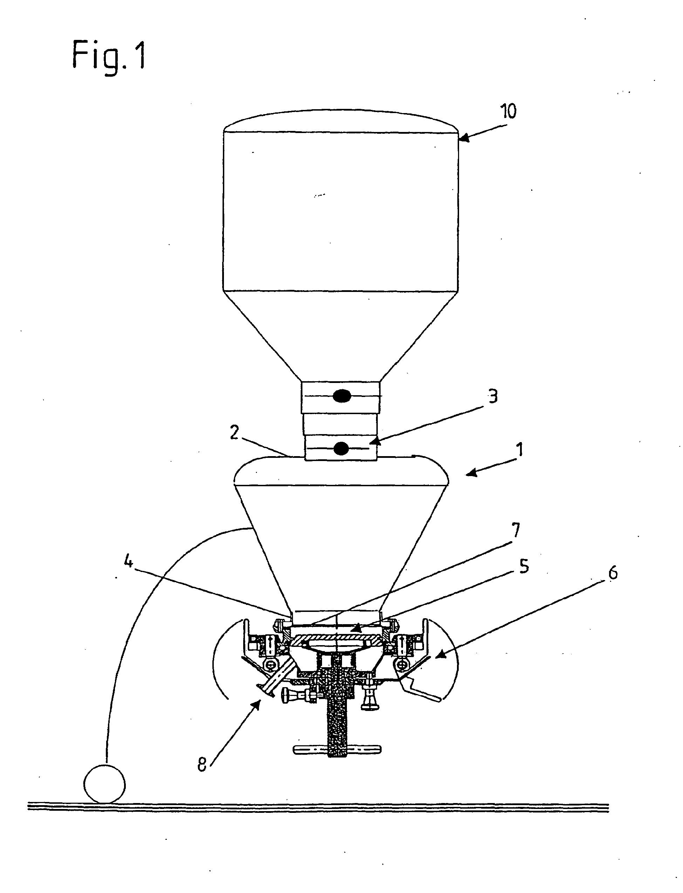

Im folgenden wird anhand der beiliegenden Zeichnungen ein Ausführungsbeispiel der Erfindung beschrieben. Es zeigen
- Fig. 1: eine schematische Darstellung eines Transportbehälters für sterile Verschlussteile
- Fig. 2: den Transportbehälter in drei aufeinanderfolgenden Verfahrensschritten

Der in der Figur gezeigte Transportbehälter 1 für sterile Verschlussteile, wie Kappen, Stopfen etc., besitzt eine für solche Behälter übliche konische Form und bleibt während aller Verfahrensschritte in der gezeigten, sich nach unten verjüngenden Position. An seiner breiten Oberseite 2 besitzt der Behälter 1 eine Einfüllöffnung 3 mit einer Einfüllvorrichtung, durch welche die sterilen Verschlussteile aus einem Behandlungsbehälter 10 in den Transportbehälter 1 abgelassen werden.

Bevor die Verschlussteile in den Transportbehälter transferiert werden, muss der Transportbehälter gereinigt und sterilisiert werden. Der Transportbehälter ist deshalb mit einem Medienvorlauf ausgerüstet. Durch diesen kann Reinigungsmittel für die Reinigung und Dampf für die Sterilisierung in den Transportbehälter gebracht werden.

An seiner Unterseite 4 ist der Transportbehälter 1 mit einem sog. Rapid Transfer Port 5 versehen. Der Rapid Transfer Port ist mit einer zusätzlichen Abdeckung 6 ausgerüstet. Solche mit einer Abdeckung versehene Rapid Transfer Ports sind beispielsweise unter dem Fabrikat "La Calhène" handelsüblich. Die Abdeckung 6 erlaubt es, den Port zu reinigen und unter Druck zu sterilisieren. Zusätzlich werden durch einen Medienauslass 8 in der Abdeckung die Medien, welche für die Reinigung und Sterilisierung des Transportbehälters benötigt wurden, abgeführt.

Die Abdeckung 6 ist zusätzlich mit einer Vorrichtung ausgerüstet, welche es erlaubt den Portdeckel 7 zu öffnen. Sowohl das Öffnen als auch das Verschliessen des Portdeckels kann automatisch mit einem geeigneten Antrieb oder manuell erfolgen. Mit dieser Einrichtung ist somit eine bakteriologisch und sterilisationstechnisch einwandfreie Reinigung und Sterilisation aller Teile gewährleistet, welche mit den Verschlussteilen in Berührung kommen. Sämtliche mit dem Produkt in Berührung kommenden Teile sind sogenannt durchgereinigt und durchsterilisiert. Nach der Reinigung bzw. nach der Sterilisation und der nachfolgenden Trocknung wird der Portdeckel wieder geschlossen. Damit ist der Innenteil des Transportbehälters einwandfrei gereinigt und steril.

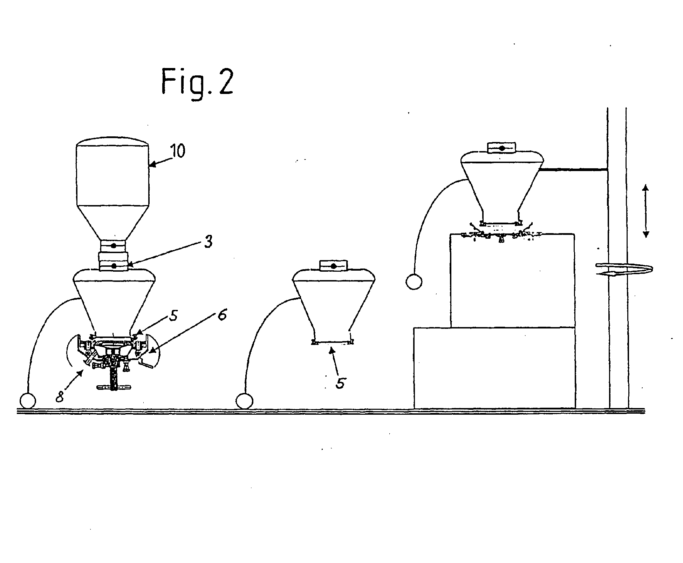

Wie in Fig. 2 gezeigt ist der Transportbehälter 1 mit seiner Einfüllöffnung in einem ersten Verfahrensschritt mit einem Behandlungsbehälter 10 verbunden, um die in letzterem gereinigten und sterilisierten Verschlussteile zu übernehmen. Anschliessend können die Verschlussteile im Transportbehälter gelagert werden, bis sie zur weiteren Verwendung in einem dritten Schritt benötigt werden. Dafür wird der Transportbehälter mit dem Rapid Transfer Port 5 an einer entsprechenden nachgeordneten Einrichtung, beispielsweise einer Abfüllanlage, angedockt.

## Patentansprüche

1. Transportbehälter (1) für den Transport steriler Produkte von einer Sterilisationseinrichtung zu einer nachgeordneten Einrichtung für die bestimmungsgemässe Verwendung der Produkte, sowie das sterile Andocken an die nachgeordnete Einrichtung, mit zwei einander gegenüber angeordneten Anschlussvorrichtungen (3, 5), von denen eine mit einem Portdeckel (7) und mit einem Rapid Transfer Port zum Andocken des Transportbehälters an die nachgeordnete Einrichtung ausgerüstet ist, wobei der Rapid Transfer Port (5) mit einer Abdeckung (6) ausgerüstet ist, , **dadurch gekennzeichnet, dass** die dem Rapid Transfer Port gegenüber liegende Anschlussvorrichtung (3) eine Einfüllvorrichtung für die Produkte ist und dass die Abdeckung (6) des Rapid Transfer Port (5) mit einer Öffnungsvorrichtung zum Öffnen des Portdeckels (7) und mit einem Medienrücklauf (8), durch welchen bei geöffnetem Portdeckel (7) Reinigungs- und Sterilisationsmedien ableitbar sind, versehen ist.

## Claims

1. Transport container (1) for transporting sterile products from a sterilization device to a downstream device for use of the products in the intended manner, and the sterile docking to the downstream device, comprising two connection apparatuses (3, 5) which are arranged opposite one another and one of which is equipped with a port cover (7) and with a rapid transfer port for docking the transport container to the downstream device, the rapid transfer port (5) being equipped with a covering (6), **characterized in that** the connection apparatus (3) opposite the rapid transfer port is a filling apparatus for the products and **in that** the covering (6) of the rapid transfer port (5) is provided with an opening apparatus for opening the port cover (7) and with a media return (8) through which cleaning and sterilization media can be discharged when the port cover (7) is opened.

## Revendications

1. Conteneur de transport (1) pour le transport de produits stériles d'un dispositif de stérilisation vers un dispositif suivant pour une utilisation des produits selon les dispositions, ainsi que l'arrimage stérile au dispositif suivant, avec deux dispositifs de raccordement (3, 5) disposés l'un à l'opposé de l'autre, dont l'un est muni d'un couvercle de port (7) et d'un Rapid Transfer Port pour l'arrimage du conteneur de transport au dispositif suivant, où le Rapid Transfer Port (5) est muni d'un couvercle (6), **caractérisé en ce que** le dispositif de raccordement (3) situé à l'opposé du Rapid Transfer Port est un dispositif de remplissage pour les produits, et **en ce que** le couvercle (6) du Rapid Transfer Port (5) est muni d'un dispositif d'ouverture pour l'ouverture du couvercle de port (7) et d'un reflux de médium (8), à travers lequel des médias de nettoyage et de stérilisation peuvent être déviés lorsque le couvercle de port (7) est ouvert.
